# EUROPEAN PATENT APPLICATION

(11) **EP 4 843 401 A1**
(43) Date of publication of application: **25.11.2026**
(21) Application number: 25856964.9
(22) Date of filing: 20.08.2025
(51) Int. Cl.: A23L 33/10, A61K 35/57, A61P 21/00, A61P 29/00

(54) **MUSCLE FIBER DIFFERENTIATION PROMOTER AND FOOD COMPOSITION FOR PROMOTING MUSCLE FIBER DIFFERENTIATION**

(30) Priority: 20.08.2024 JP 2024139319
(71) Applicant: M-Style Holdings Co., Ltd., Fukuoka-shi, Fukuoka 810-0021 (JP); Miranest Co., Ltd., Fukuoka-shi, Fukuoka 810-0021 (JP)
(72) Inventor: NAGANE Masaki, Sagamihara-shi, Kanagawa 252-5201 (JP); YAMASHITA Tadashi, Sagamihara-shi, Kanagawa 252-5201 (JP); FUJIKI Yuha, Fukuoka-shi, Fukuoka 810-0021 (JP); KONISHI Yoshinari, Fukuoka-shi, Fukuoka 810-0021 (JP); INATOMI Mikiya, Fukuoka-shi, Fukuoka 810-0021 (JP)
(74) Representative: Valente Cioncoloni, Andrea
(86) International application number: PCT/JP2025/029259
(87) International publication number: WO 2026/042831

(57) **Abstract**

The present invention aims to provide a more effective muscle repair enhancement promoter and the like than conventional ones.

A muscle fiber differentiation promoter containing a component contained in edible bird's nest as an active ingredient and promoting differentiation of cells into muscle fibers. In particular, by obtaining active ingredients from edible bird's nest through hydrolysis treatment, the active ingredients contained in edible bird's nest can be obtained without loss compared to conventional methods of extraction through heat treatment. Therefore, it becomes possible to enhance the effect of promoting muscle repair enhancement by the active ingredients contained in edible bird's nest.

## Description

### [Technical Field]

The present invention relates to a muscle fiber differentiation promoter and a food composition for promoting muscle fiber differentiation.

### [Background Art]

Muscle damage and repair is an important physiological process caused by various factors such as exercise, injury, and aging. Muscle damage is accompanied by destruction of muscle fibers, activation of inflammatory responses, and initiation of repair processes. The repair process includes activation and differentiation of muscle satellite cells, reconstruction of the extracellular matrix, and formation of new muscle fibers (Non-Patent Literature 1).

In recent years, research on the effects of nutritional supplements and specific food components on muscle repair has been increasing (Non-Patent Literature 2). In particular, it has been shown that proteins, amino acids, antioxidants, and anti-inflammatory substances may promote muscle repair (Non-Patent Literature 3). These components act through reduction of inflammation, activation of muscle satellite cells, alleviation of oxidative stress, and regeneration of muscle fibers (Non-Patent Literature 4).

Among these, animal proteins and amino acids extracted from milk components such as whey protein have been considered effective as muscle repair and enhancement promoters.

It is also known that sialic acid has an improvement effect on sarcopenia, a condition in which muscle mass decreases with aging and muscle strength and physical function decline (Patent Literature 1). In addition, it is known that sialic acid derived from bird's nest activates cell growth factors (Patent Literature 2). It is also known that oral administration of sialic acid to model mice improved the contractile force of skeletal muscle (Non-Patent Literature 5).

### [Prior Art Literature]

### [Patent Literature]

[Patent Literature 1] JP 2018-030813 A
[Patent Literature 2] JP 2009-234980 A

### [Non-Patent Literature]

[Non-Patent Literature 1] Brack AS, Rando TA. Tissue-specific stem cells: lessons from the skeletal muscle satellite cell. Cell Stem Cell. 2012;10(5):504-514.
[Non-Patent Literature 2] Phillips SM, Van Loon LJ. Dietary protein for athletes: from requirements to optimum adaptation. J Sports Sci. 2011;29 Suppl 1: S29-S38.
[Non-Patent Literature 3] Cannon JG, Orencole SF, Fielding RA, et al. Acute phase response in exercise: interaction of age and vitamin E on neutrophils and muscle enzyme release. Am J Physiol. 1990;259(6 Pt 2): R1214-R1219.
[Non-Patent Literature 4] Powers SK, Jackson MJ. Exercise-induced oxidative stress: cellular mechanisms and impact on muscle force production. Physiol Rev. 2008;88(4):1243-1276.
[Non-Patent Literature 5] Diego Moreno-Perez et al. Clinical Neurology, 2009, Vol.49, No.11, pp.852-855.
[Non-Patent Literature 6] Diego Moreno-Perez et al. Effect of a Protein Supplement on the Gut Microbiota of Endurance Athletes: A Randomized, Controlled, Double-Blind Pilot Study, Nutrients. 2018 Mar; 10(3): 337.

### [Summary of Invention]

### [Technical Problem]

However, there have been concerns that animal proteins and amino acids extracted from milk components such as whey protein may disrupt the balance of the intestinal microbiota and affect physical condition, skin health, and immunity. (Non-Patent Literature 6).

Therefore, an object of the present invention is to provide a muscle repair and enhancement promoter and the like that can suppress adverse effects on physical condition.

### [Solution to Problem]

A first aspect of the present invention is a muscle repair and enhancement promoter that promotes repair or enhancement of muscle, containing a component contained in bird's nest as an active ingredient.

A second aspect of the present invention is the muscle repair and enhancement promoter of the first aspect, wherein the active ingredient is a component contained in an aqueous solution obtained by hydrolyzing bird's nest without undergoing extraction treatment.

A third aspect of the present invention is the muscle repair and enhancement promoter of the second aspect, wherein the hydrolyzing is performed such that the proportion of products derived from bird's nest having a molecular weight of 6000 or less falls within the range of 40-60%.

A fourth aspect of the present invention is the muscle repair and enhancement promoter of the second aspect, wherein the hydrolyzing is performed by preparing a solution such that the weight percent of bird's nest falls within the range of 5-15 ww%.

A fifth aspect of the present invention is the muscle repair and enhancement promoter of any one of the first to fourth aspects, which promotes repair or enhancement of muscle by promoting differentiation of cells into muscle fibers.

A sixth aspect of the present invention is a food composition for promoting muscle repair and enhancement that promotes repair or enhancement of muscle, containing a component contained in bird's nest as an active ingredient.

The present invention may also be regarded as a muscle fiber differentiation promoter, comprising a component contained in edible bird's nest as an active ingredient, and wherein the muscle fiber differentiation promoter promotes differentiation of cells into muscle fibers. The present invention may also be regarded as a food composition for promoting muscle fiber differentiation, comprising a component contained in edible bird's nest as an active ingredient, and wherein the food composition promotes differentiation of cells into muscle fibers.

### [Advantageous Effects of Invention]

Conventionally, the effect of the active ingredient contained in bird's nest on muscle repair or enhancement was not known. However, each aspect of the present invention was discovered by the present inventors who verified and found that the component contained in bird's nest has such effect.

Bird's nest is known to maintain or improve the environment of the intestinal microbiota. Therefore, according to each aspect of the present invention, by using bird's nest as a muscle repair and enhancement agent, it becomes possible to provide a muscle repair and enhancement promoter and the like that can maintain or improve the environment of the intestinal microbiota.

Furthermore, according to the second aspect of the present invention, it becomes possible to obtain the active ingredient without undergoing extraction treatment that may cause some active ingredients to outflow from the bird's nest.

Furthermore, according to the third and fourth aspects of the present invention, it becomes easy to produce a muscle repair and enhancement promoter that effectively contains the active ingredients contained in bird's nest.

Furthermore, according to the fifth aspect of the present invention, the present inventors found that the active ingredient contained in bird's nest promotes differentiation of cells into muscle fibers.

Patent Literature 1 describes that sialic acid had an improvement effect on sarcopenia. However, there is no mention of the action of promoting differentiation of cells into muscle fibers. From the mere description in Patent Literature 1 that there was an improvement effect on sarcopenia, it is completely unclear through what specific pathway the improvement was achieved and what the mechanism of action is. Patent Literature 2 and Non-Patent Literature 5 also contain no description or suggestion of promoting muscle fiber differentiation.

Here, as methods for improving sarcopenia, there exist numerous methods including at least improvement of mitochondrial function, control of inflammation and improvement of anabolic resistance, maintenance of neuromuscular junction (NMJ) and motor units, improvement of angiogenesis and capillary density (microcirculation), improvement of autophagy/proteostasis, nutritional assimilation (HMB/creatine, etc.), and improvement of endocrine substances (testosterone, etc.). Among these, chronic inflammation is one of the main causes of sarcopenia.

As described above, numerous methods are conceivable as improvement methods for sarcopenia. Among these, which method is effective for a specific patient depends on the symptoms of that patient. Furthermore, there is no description or suggestion in Patent Literature 1, Patent Literature 2, or Non-Patent Literature 5 regarding what specific mechanism of action bird's nest can be expected to have an improvement effect on sarcopenia.

For example, if the cause of a patient's sarcopenia is deterioration of the structure or function of the neuromuscular junction (NMJ), which is one of the main causes, measures such as improvement of the patient's exercise habits, nutritional status, and neural regulation are necessary for NMJ preservation. Furthermore, if aging muscle performance is declining due to sparse capillaries, angiogenesis treatment is considered to be effective. Thus, even though it is called sarcopenia in general terms, the specific causes are various, and different treatments corresponding to those causes are effective for improvement.

The muscle fiber differentiation promoter or food composition for promoting muscle fiber differentiation according to the present invention is clearly specified to be effective for patients who require muscle fiber differentiation.

The finding that the active ingredient contained in bird's nest specifically exhibits the above effects was first discovered through research by the inventors of the present application, and is clearly different from the inventions described in Patent Literatures 1 and 2 and Non-Patent Literature 5.

### [Brief Description of Drawings]

[FIG. 1] A diagram showing the results of Western blot analysis in C2C12 cells.
[FIG. 2] A diagram showing the measurement results of reactive oxygen species (ROS) in Caco-2 cells.
[FIG. 3] A diagram showing the effects of edible bird's nest extract specimens in a mouse muscle injury model, showing (a) recovery of gait, (b) enhancement of grip strength, and (c) increase in muscle cross-sectional area.
[FIG. 4] A diagram showing the effects of edible bird's nest extract specimens in a mouse muscle injury model, showing suppression of the expression of genes of inflammatory cytokine: (a) TNFα, (b) IL-6, and (c) IFNβ1.

### [DETAILED DESCRIPTION OF THE INVENTION]

Hereinafter, embodiments of the present invention will be described in detail with reference to the drawings. It should be noted that the examples of the present invention are not limited to the contents described below.

### [Example 1]

The inventors of the present application examined the anti-inflammatory effects and muscle repair promoting effects of edible bird's nest hydrolyzed extract in a muscle injury model. Furthermore, the effects of edible bird's nest hydrolyzed extract were verified relating to the antioxidant effects, direct effects on muscle cells, and indirect effects via the intestinal tract.

First, the materials used for verification and the verification methods will be described.

### <Preparation of Edible Bird's Nest Extract>

The edible bird's nest extract used was prepared by M-Style Holdings Co., Ltd., one of the applicants of the present application. As a preparation procedure, first, RO membrane-treated water was used for Malaysian swiftlet nests (specimens) that had been dried and adjusted to a moisture content of 10% or less, and the specimens were mixed and soaked so that the specimen concentration was in the range of 5-15 ww%. Thereafter, hydrolysis treatment was performed so that the proportion of molecular weight 6000 or less was in the range of 40-60%.

Thereafter, the residue was removed from the finished hydrolysate using a 100-mesh sieve. Furthermore, centrifugation (10000 rpm/20 min) was performed to spin down further residue, and the supernatant liquid was collected as the extract specimen

### (EBN).

EBN was centrifuged at 10,000 rpm for 10 minutes, and solutions were prepared from the supernatant at concentrations of 0.5, 1.0, and 1.5 mg/mL using 1×PBS as a solvent. The obtained solutions were stored at -20°C and thawed as appropriate when used.

### <Preparation of H₂O₂ Solution>

H₂O₂ (FUJIFILM Wako Pure Chemical Corporation, Osaka, Japan) was prepared to 100 mM using sterile water, filter-sterilized through a 0.22 µm filter (Merck Millipore, Munich, Germany), stored at -20°C, and thawed as appropriate for use.

Next, the measurement methods and analysis methods will be described.

### <Measurement of Antioxidant Activity (H-ORAC Method)>

The measurement samples were prepared by dissolving dried and ground powder of specimens before hydrolysis treatment (P) or freeze-dried powder of EBN after hydrolysis treatment (EBNFD) in pure water.

Antioxidant activity of the measurement samples was measured by the H-ORAC method. The results were expressed as H-ORAC values and calculated as Trolox equivalents.

### <Cultivation of C2C12 Cells>

In this study, the mouse skeletal muscle-derived myoblast cell line C2C12 was used as a skeletal muscle cell model. C2C12 cells were subcultured in Dulbecco's Modified Eagle Medium (DMEM) (Nissui pharmaceutical, Tokyo, Japan) supplemented with 10% heat-inactivated Fetal Bovine Serum (FBS) (complement inactivated by heating at 56°C in a constant temperature bath for 30 minutes), 4 mM L-glutamine (FUJIFILM Wako), 100 U/mL penicillin (FUJIFILM Wako), and 100 µg/mL streptomycin (Meiji seika pharma, Tokyo, Japan) at 37°C in the presence of 5% CO₂.

C2C12 cells were seeded in 6-well plates at a concentration of 2.0×10⁵ cells/well, and after 48 hours, the medium was replaced with DMEM medium containing 2% Horse Serum (HS) (Thermo Fisher Scientific, Inc., Waltham, MA, USA) with a final concentration of 1.5 µg/mL EBN to induce differentiation. For the control, 1×PBS equivalent to the amount of EBN added was added. Thereafter, the medium was exchanged and EBN was added every 2 days, and the cells were cultured for 7 days. To investigate the effect of EBN during muscle injury, cells were collected on days 3, 5, and 7, wherein cells had been treated in the presence or absence of a final concentration of 200 µM H₂O₂ 24 hours before cell collection.

### <Western Blot Analysis>

C2C12 myotube cells were lysed with RIPA Buffer (182-02451, FUJIFILM Wako) supplemented with Protease Inhibitor Cocktail Set III DMSO Solution (EDTA free) (×100, 163-26061, FUJIFILM Wako), and protein concentration was measured using Micro BCA Protein Assay Kit (Thermo Fisher Scientific). A total of 20-30 µg of protein was loaded onto a 10% SDS-PAGE gel, and after protein separation by gel electrophoresis, it was transferred to Amersham Hybond P PVDF 0.45 (GE Healthcare UK Ltd., Buckinghamshire, UK).

After transfer, blocking was performed with 5% skim milk in 0.1% TBS-T at room temperature for 1 hour. Primary antibodies were diluted in blocking buffer and shaken overnight at 4°C. The following primary antibodies were used: β-Tubulin (1:1000; 2128, Cell Signaling Technology; CST, Danvers, MA, USA), MyoD (1:5000; 18943-1-AP, Proteintech, Rosemont, IL, USA), and Myosin Heavy Chain (1:1000; MAB4470, R&D Systems, Minneapolis, MN, USA).

After primary antibody reaction, HRP-conjugated anti-Rabbit secondary antibody (1:2000; 7074, CST) or anti-Mouse secondary antibody (1:2000; 7076, CST) was diluted in blocking buffer and shaken at room temperature for 1 hour. After secondary antibody reaction, chemiluminescence was performed using ImmunoStar Zeta (FUJIFILM Wako), and band detection was performed using Lumino Graph I (ATTO, Tokyo, Japan). Quantitative analysis of protein expression was performed using Image J software.

### <Cultivation of Caco-2 Cells>

In this study, the human colon cancer-derived cell line Caco-2 was used as a human intestinal epithelial cell model. Caco-2 cells were subcultured in DMEM medium supplemented with 10% heat-inactivated FBS, 4 mM L-glutamine, 100 U/mL penicillin, and 100 µg/mL streptomycin at 37°C in the presence of 5% CO₂.

Caco-2 cells were seeded in 6-well plates at a concentration of 2.0×10⁵ cells/well, and after 24 hours, the medium was replaced with DMEM medium containing 10% FBS with final concentrations of 0, 0.5, 1.0, and 1.5 µg/mL EBN. For the control, 1×PBS equivalent to the amount of EBN added was added. Cells 24 hours after EBN addition were used for measurement of intracellular reactive oxygen species (ROS). Oxidative stress was induced by adding a final concentration of 200 µM H₂O₂ simultaneously with EBN.

### <Measurement of Intracellular Reactive Oxygen Species (ROS) in Caco-2 Cells>

Intracellular ROS in Caco-2 cells was measured using the cell-permeable reagent 2,7-dichlorodihydrofluorescein diacetate (DCFDA) (Abcam, Cambridge, UK). After diffusion into cells, DCFDA is deacetylated by intracellular esterases to become a non-fluorescent compound. It is then oxidized by ROS to become 2', 7'-dichlorofluorescein (DCF). DCF is highly fluorescent and is detected by fluorescence spectroscopy at excitation/emission of 485 nm/535 nm.

DCFDA solution at a final concentration of 20 µM was added to Caco-2 cells and incubated at 37°C for 30 minutes. After removing the DCFDA solution, cells were suspended in 1×Buffer and analyzed using a flow cytometer (CytoFlex, Beckman Coulter, Brea, CA, USA). Results were expressed as relative mean fluorescence intensity (MFI) compared to control.

### <Mouse Muscle Injury Model>

200 nmol of Cardiotoxin (CTX, LATOXAN, France) was administered intramuscularly to the left gastrocnemius muscle of C57BL/6N mice (male, 10 weeks old) in a volume of 50 µL using a 29G Myjector. Physiological saline was administered intramuscularly to the contralateral limb. EBN (80 mg/mouse) was orally administered immediately after CTX administration, and thereafter administered at 24-hour intervals. Motor function was evaluated using the Tarlov score 1-14 days after CTX administration (0: no voluntary movement, 1: barely perceptible movement, 2: leg moves but no coordination with contralateral limb and no weight bearing, 3: alternating stepping and propulsive movements with no weight bearing, 4: weight bearing, 5: walking with mild impairment, 6: normal walking). Furthermore, grip strength was measured 7 days after administration using a grip strength meter (Bioseb) and corrected for body weight. In addition, tissue materials were collected after euthanasia following fecal collection.

### <Histological Analysis of Muscle Tissue>

The collected muscle tissue was fixed in neutral buffered formalin and sectioned at 2 µm after paraffin embedding. Tissue sections were deparaffinized, hydrated, and stained with HE, then imaged using a KEYENCE all-in-one microscope. Muscle cross-sectional area was analyzed using microscope-attached software.

### <Gene Expression Analysis of Muscle Tissue>

RNAiso plus (Takara Bio, Japan) was added to the collected muscle, which was then homogenized using a bead homogenizer. Chloroform was added to the extract solution after homogenization for phase separation. Total RNA was purified from the aqueous layer after separation using the FavorPrep Tissue Total RNA Mini Kit (Favorgen, Taiwan). 500 ng of Total RNA was aliquoted and reverse transcribed using ReverTra Ace qPCR RT Master Mix with gDNA remover (Toyobo life science) to prepare cDNA. FS essential DNA Green master (Roche) and gene-specific primers were mixed with cDNA, and changes in fluorescence accompanying gene amplification were measured using the LightCycler96 (Roche). Relative gene expression levels were calculated using the ΔΔCt method.

### <Statistical Analysis>

Statistical analysis was performed using the Tukey HSD test, and p < 0.05 was considered statistically significant. Statistical analysis for mouse experiments was performed using GraphPad Prism10 with multiple group comparisons by the Tukey method.

The measurement results and analysis results are described below.

### <Comparison of Antioxidant Activity Before and After Hydrolysis>

Antioxidant activity was measured by the H-ORAC method for the dried powder of the specimen before hydrolysis treatment (P) and the freeze-dried powder of EBN after hydrolysis treatment (EBNFD). When the Trolox equivalent H-ORAC values per 1 g of samples were calculated, EBNFD showed a significantly higher H-ORAC value compared to P (Table 1).

**[Table 1]**

| Table 1 | H-ORAC Values of Swallow Nests Before and After Hydrolysis |
|---|---|
| | H-ORAC^{Value}(µmol) |
| P | 0.9 |
| EBNFD | 231 |

| | |
|---|---|
| Per 1 gram of sample (Trolox equivalent) | |

### <Effect of EBN on C2C12 Cell Differentiation>

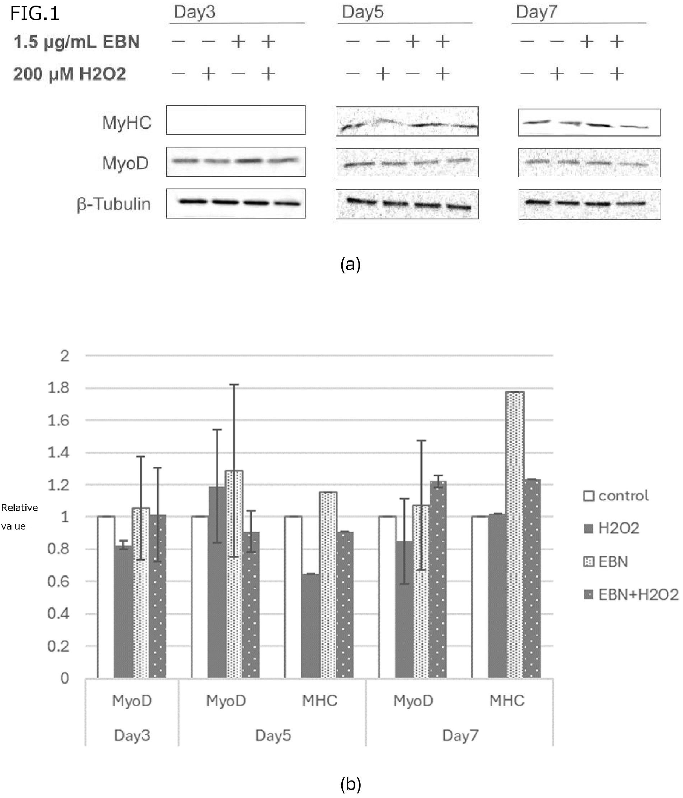

In C2C12 cells supplemented with EBN, the expression level of Myosin Heavy Chain (MHC) increased, and differentiation was promoted (Fig. 1(a) and Fig. 1(b)).

### <Effect of EBN on H₂O₂-Induced Oxidative Stress in Caco-2 Cells>

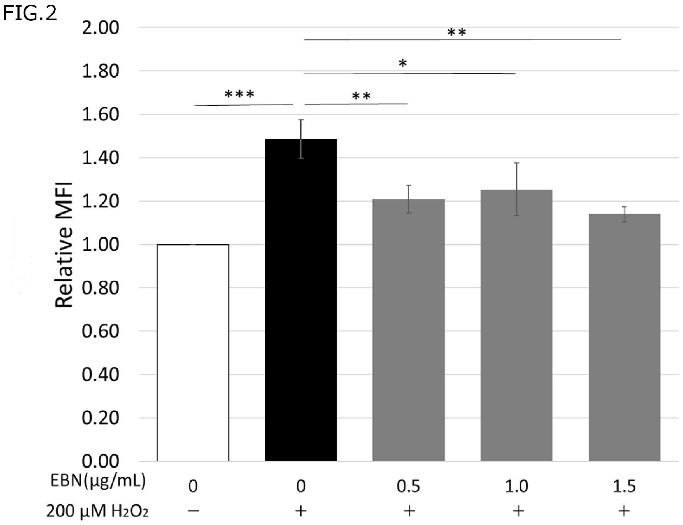

ROS levels in Caco-2 cells subjected to oxidative stress induction using H₂O₂ were reduced by the addition of EBN (Fig. 2).

### <Effect of EBN in Mouse Muscle Injury Model>

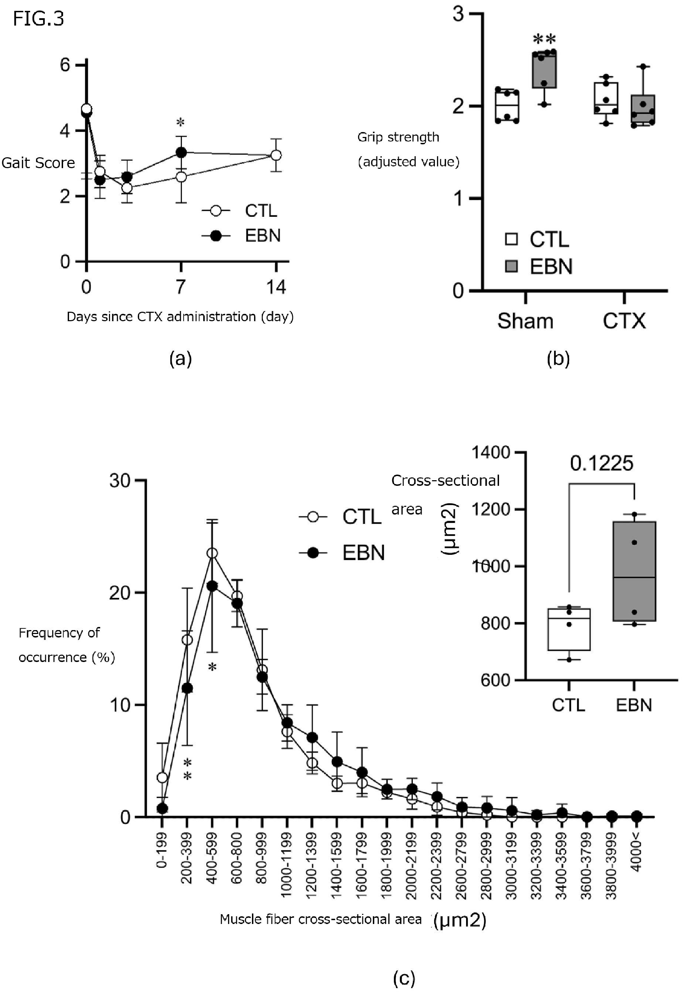

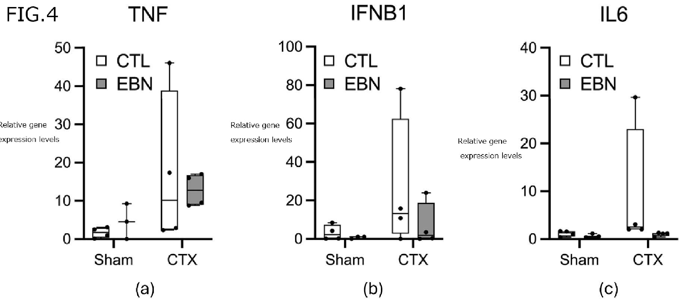

When EBN was administered to mice with muscle injury, recovery from muscle injury was promoted (Fig. 3(a)). In addition, grip strength of the contralateral limb was enhanced (Fig. 3(b)). Histological analysis showed that EBN administration reduced the number of immature muscle fibers and increased muscle cross-sectional area (Fig. 3(c)). As shown in Fig. 4, gene expression analysis revealed that oral administration of EBN suppressed the expression of genes of inflammatory cytokine; (a) TNFα, (b) IL-6, and (c) IFNβ1. From the above, it was suggested that EBN has muscle repair promoting and muscle strength enhancing effects.

Based on the above results, the present inventors suggested that edible bird's nest hydrolysate extract (EBN) has muscle repair promoting and anti-inflammatory effects. First, in the evaluation of antioxidant activity by the H-ORAC method, EBN after hydrolysis showed remarkable antioxidant activity, which may contribute to reducing oxidative stress after muscle injury.

Furthermore, in promoting C2C12 cell differentiation, an increase in MHC expression level by EBN addition was confirmed, indicating that EBN acts directly on muscle and promotes muscle repair.

Furthermore, since EBN reduced H₂O₂-induced oxidative stress in Caco-2 cells, it was suggested that the antioxidant effect of EBN also contributes to indirect muscle repair promotion through the intestinal tract. The mechanism by which improvement of the intestinal environment reduces systemic inflammatory responses and promotes muscle repair is expected to be further elucidated in future research.

In the mouse muscle injury model, EBN administration promoted recovery from muscle injury and enhanced muscle strength was observed. In particular, the reduction in the number of immature muscle fibers, increase in muscle cross-sectional area, and suppression of inflammatory cytokine gene expression were confirmed, suggesting that EBN supports muscle repair in a multifaceted manner. The anti-inflammatory effect of EBN may suppress excessive inflammatory responses during the muscle repair process and promote normal repair.

From the above results, it was demonstrated that edible bird's nest hydrolysate extract (EBN) may effectively promote repair after muscle injury and muscle strength enhancement through direct action on muscle cells and indirect action via intestinal cells. Based on the above results, the utilization of active ingredients contained in EBN for muscle repair enhancement promoters, food compositions for promoting muscle repair enhancement, muscle fiber differentiation promoters, and food compositions for promoting muscle fiber differentiation is expected.

## Claims

1. A muscle fiber differentiation promoter, comprising:
a component contained in edible bird's nest as an active ingredient, and
wherein the muscle fiber differentiation promoter promotes differentiation of cells into muscle fibers.

2. A food composition for promoting muscle fiber differentiation, comprising:
a component contained in edible bird's nest as an active ingredient, and
wherein the food composition promotes differentiation of cells into muscle fibers.
